# FASCICULE DE BREVET EUROPEEN

(11) **EP 2 725 961 B1**
(45) Date de publication et mention de la délivrance du brevet: **02.09.2020**
(21) Numéro de dépôt: 12730914.4
(22) Date de dépôt: 25.06.2012
(51) Int. Cl.: A61B 1/00, A61B 17/00

(54) **INSTRUMENT ENDOSCOPIQUE A PIED D'APPUI**
ENDOSKOPISCHES INSTRUMENT MIT STÜTZFUSS
ENDOSCOPIC INSTRUMENT WITH SUPPORT FOOT

(30) Priorité: 29.06.2011 FR 1155827
(43) Date de publication de la demande: 07.05.2014
(73) Titulaire: Université Pierre et Marie Curie (Paris 6), 75005 Paris (FR); Centre National de la Recherche Scientifique, 75016 Paris (FR); Mauna Kea Technologies, 75010 Paris (FR); Endocontrol, 38700 La Tronche (FR)
(72) Inventeur: ROSA, Benoît, F-92240 Malakoff (FR); HERMAN, Benoît, B-1200 Woluwe-Saint-Lambert (BE); SZEWCZYK, Jérôme, F-95510 Vienne-en-Arthies (FR); MOREL, Guillaume, F-75013 Paris (FR); VIDAL, Clément, F-38000 Grenoble (FR); HENRI, Patrick, F-92270 Bois Colombes (FR); LACOMBE, François, F-92370 Chaville (FR); LOPEZ, Jérôme, F-13100 Aix-en-Provence (FR)
(74) Mandataire: Lavialle, Bruno François Stéphane
(86) Numéro de dépôt international: PCT/EP2012/062264
(87) Numéro de publication internationale: WO 2013/000873

(56) Documents cités:
- EP-A2- 1 946 707
- US-A1- 2005 137 453
- US-A1- 2006 254 603
- US-A1- 2011 060 227

## Description

L'invention est relative à un instrument endoscopique à pied.

### ARRIERE PLAN DE L'INVENTION

Les instruments endoscopiques sont introduits dans le corps du patient, par exemple dans la cavité intra-abdominale, par l'intermédiaire d'une voie naturelle ou d'une voie artificielle, par exemple une canule, en vue d'intervenir sur un organe interne. Il peut s'agir d'effectuer une biopsie de l'organe, ou d'y enfoncer une sonde intra-tissulaire à un endroit précis de l'organe interne.

Il peut encore s'agir d'inspecter une portion de la surface de l'organe interne. L'instrument endoscopique est alors muni à son extrémité distale d'un outil, par exemple une caméra ou une sonde ultrasonore. Selon le cas, il convient d'assurer un positionnement et une immobilité parfaite vis-à-vis de l'organe interne, ou encore il convient alors d'opérer un déplacement relatif de l'extrémité distale de l'instrument endoscopique par rapport à l'organe interne, de façon sensiblement parallèle à une surface externe de celui-ci, par exemple un mouvement de balayage.

Pour effectuer un tel déplacement, le praticien déplace l'instrument endoscopique de façon manuelle ou avec l'aide d'une assistance robotisée en agissant sur la partie extra-corporelle de l'instrument endoscopique. Cependant, des tels mouvements sont peu précis et ne tiennent compte ni des mouvements du patient induisant un mouvement de la voie d'accès, ni des mouvements propres de l'organe dans le corps du patient.

Il a été proposé d'utiliser des moyens d'immobilisation de l'organe, en l'occurrence un outil de forme qui est plaqué fortement sur l'organe pour tenter d'immobiliser celui-ci. Cependant, l'immobilité de l'organe n'est pas garantie. En outre, ce genre de dispositif d'immobilisation est encombrant et invasif.

Il a encore été proposé de réaliser un asservissement de la position de l'extrémité distale de l'instrument endoscopique relativement à l'organe interne au moyen d'une caméra embarquée, l'asservissement visant à annuler les déplacements relatifs de l'extrémité distale de l'instrument endoscopique relativement à l'organe interne. Ce procédé nécessite la mise en œuvre d'une acquisition vidéo en temps réel ainsi que la mise en œuvre d'un algorithme d'asservissement récursif et adaptatif complexe.

Il a encore été proposé de réaliser un asservissement en effort, consistant à annuler la composante périodique des efforts appliqués à l'outil par l'organe, imputable aux mouvements physiologiques de l'organe interne.

Il a encore été proposé d'équiper l'extrémité distale de l'instrument endoscopique de moyens de déplacement commandés disposés entre l'instrument endoscopique et l'outil pour opérer un déplacement de l'outil par rapport à l'instrument endoscopique au moins selon des directions transverses à un axe longitudinal de l'extrémité de l'instrument endoscopique.

Selon un premier mode de mise en œuvre, l'instrument endoscopique est immobilisé alors que l'outil est à distance de l'organe, et les moyens de déplacement sont commandés pour effectuer un mouvement relatif de l'outil par rapport à l'organe. Cependant, l'immobilisation de l'instrument endoscopique peut générer des contraintes sur la voie d'accès du patient, qui peuvent incommoder celui-ci. En outre, les mouvements propres de l'organe empêchent tout déplacement ou positionnement précis de l'outil vis-à-vis de l'organe.

Selon un deuxième mode de mise en œuvre, l'outil est amené au contact de l'organe, et l'instrument endoscopique est laissé libre de bouger avec le patient. Cependant, une adhérence peut se manifester entre l'outil et l'organe qui provoque une résistance au déplacement de l'outil, empêchant tout mouvement de celui-ci vis-à-vis de l'organe ou au contraire donnant lieu à des glissements brusques et non contrôlés.

Il est également connu du document US 2011/0060227 d'équiper l'extrémité distale de l'instrument endoscopique d'un pied destiné à venir en appui sur l'organe interne. Des fils à mémoire de forme traversant entièrement l'instrument endoscopique permettent de déplacer l'outil alors que le pied est en appui contre l'organe interne.

Le document US2005/0137453 décrit un instrument endoscopique comportant un corps présentant une extremité distale portant un pieds comprenant une pince à deux mors, chacun des mors étant lié à deux cables liant la pince à I'extremité proximale de l'instrument. En tirant sur ces cables, il est dés lors possible d'imprimer à chaque mors un mouvement autour d'un axe de rotation.

### OBJET DE L'INVENTION

L'invention a pour objet de proposer un instrument endoscopique permettant un positionnement et/ou déplacement précis de l'outil porté par l'instrument vis-à-vis de l'organe interne à traiter.

### RESUME DE L'INVENTION

En vue de la réalisation de ce but, on propose un instrument endoscopique comportant un corps allongé présentant une extrémité distale destinée à être introduite dans le corps du patient pour venir à proximité d'un organe interne, l'extrémité distale portant un outil d'intervention sur l'organe, l'instrument endoscopique comportant un pied solidaire de l'extrémité distale destiné à venir en appui sur l'organe interne, ainsi que des moyens de déplacement commandables pour imprimer à l'outil des déplacements au moins selon des directions transverses à un axe longitudinal de l'extrémité distale de l'instrument endoscopique alors que le pied est en appui contre l'organe interne. Selon l'invention, les moyens de déplacement comportent au moins un actionneur qui est attelé à l'outil et qui est entièrement agencé dans le pied.

Le pied est amené en contact de l'organe interne et est appuyé sur celui-ci avec une pression suffisante pour empêcher tout mouvement relatif du pied relativement à l'organe interne. L'extrémité distale de l'instrument endoscopique suit alors les mouvements propres de l'organe interne, sans mouvement relatif vis-à-vis de ce dernier. Les moyens de déplacement sont alors commandés pour déplacer l'outil relativement à l'organe interne, l'outil étant tenu en léger retrait de la surface de l'organe interne, ou porté au contact de celle-ci sans provoquer de mouvement du pied vis-à-vis de l'organe interne. On s'assure ainsi que les mouvements physiologiques n'affectent pas la position relative de l'outil vis-à-vis de l'organe, et que les seuls mouvements de l'outil relativement à l'organe interne sont les mouvements commandés.

En outre, en plaçant l'actionneur entièrement à l'intérieur du pied, il est possible de commander des déplacements très précis de l'outil. L'actionneur peut en effet être alors de petite taille et est placé au plus près de l'outil.

De façon avantageuse, selon un mode de réalisation privilégié de l'invention, l'actionneur s'étend entre le pied et l'outil.

Le pied est amené en contact de l'organe interne et est appuyé sur celui-ci avec une pression suffisante pour empêcher tout mouvement relatif du pied relativement à l'organe interne de sorte que l'organe interne est supposé fixe dans un référentiel du pied. L'actionneur s'étendant entre le pied et l'outil, il est aisé de commander un déplacement précis et stable de l'outil relativement au pied et donc relativement à l'organe interne. Ainsi, l'agencement de l'actionneur à l'intérieur du pied permet de contrôler encore plus finement et aisément une position de l'outil relativement à l'organe interne.

### BREVE DESCRIPTION DES DESSINS

L'invention sera mieux comprise à la lumière de la description qui suit de modes particuliers de réalisation de l'invention, en référence aux figures des dessins annexés parmi lesquelles :
- la figure 1 est une représentation schématique en coupe partielle longitudinale d'un instrument endoscopique selon un premier mode particulier de réalisation de l'invention en position dans la cavité abdominale d'un patient;
- la figure 2 est une vue de face de l'extrémité distale représentée à la figure 1 ;
- les figures 3a et 3b sont des vues schématiques comparées d'un instrument connu en soi et d'un instrument selon l'invention utilisés selon une approche oblique de l'organe interne ;

- la figure 4a est une vue analogue à celle de la figure 1 d'un deuxième mode particulier de réalisation de l'instrument endoscopique de l'invention ;
- les figures 4b et 4c sont des vues analogues à celle de la figure 1 d'une variante du mode de réalisation particulier illustré à la figure 4a ;
- la figure 4d est une vue éclatée partielle de l'instrument illustré aux figures 4b et 4c ;
- la figure 5 est une vue analogue à celle de la figure 1 d'un troisième mode particulier de réalisation de l'instrument endoscopique de l'invention ;
- la figure 6 est une vue analogue à celle de la figure 1 d'un quatrième mode particulier de réalisation de l'instrument endoscopique de l'invention, le pied étant illustré en position déployée ;
- la figure 7 est une vue de l'instrument de la figure 6, le pied étant illustré en position rétractée.
- la figure 8 est une vue analogue à celle de la figure 1 d'un cinquième mode particulier de réalisation de l'instrument endoscopique de l'invention.

### DESCRIPTION DETAILLEE DE L'INVENTION

L'invention sera décrite ici en relation avec un endoscope portant une sonde d'imagerie médicale, par exemple une sonde ultra haute définition de la société Mauna Kea Technologies présentant une résolution de 1 micromètre pour un champ de vision de 240x200 micromètres et capable de prendre 12 images par seconde. Le but est de réaliser un examen visuel précis de la surface externe d'un organe interne, que l'on qualifiera ici de biopsie optique. Il est bien évident que cet exemple n'est pas limitatif.

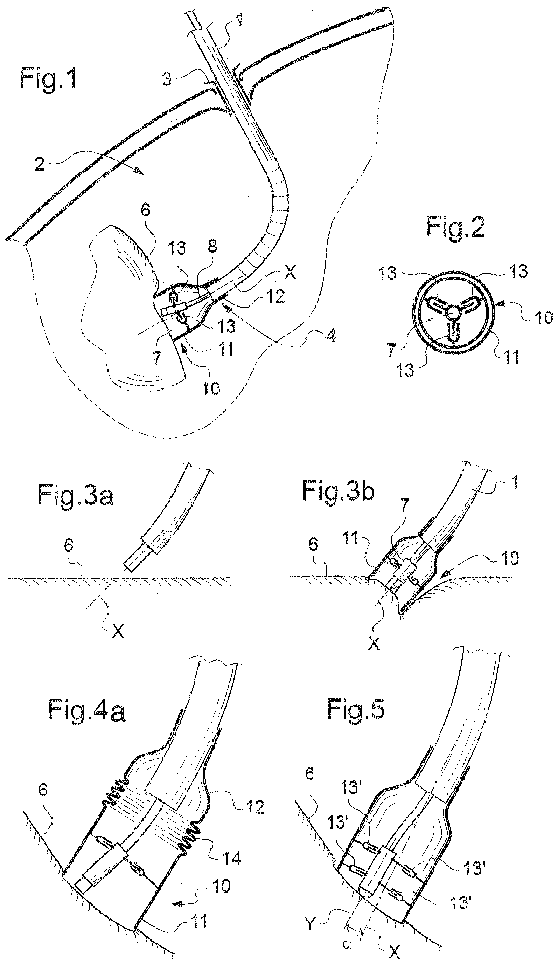

En référence aux figures 1 et 2, l'endoscope 1 comporte un corps allongé qui est introduit ici dans l'abdomen 2 d'un patient, ici par l'intermédiaire d'un trocard 3. L'endoscope 1 comporte une extrémité distale 4 qui est amenée à proximité immédiate de la surface externe d'un organe interne 6. A cet effet, l'endoscope est déformable pour amener ladite extrémité en regard de la zone d'intérêt de l'organe interne.

L'extrémité distale 4 porte ici une sonde 7 qui est reliée à son câble 8 s'étendant à l'intérieur de l'endoscope 1. Le câble 8 forme une liaison flexible de la sonde 7 à l'extrémité distale 4 de l'endoscope 1.

Selon l'invention, l'extrémité distale 4 est équipée d'un pied 10 comportant une partie d'appui 11 contre l'organe interne 6, et une partie de fixation 12 à l'extrémité distale de l'endoscope. Ici, la partie d'appui 11 et la partie de fixation 12 sont venues de matière de sorte à former un pied en forme générale de cloche. La partie de fixation 12 est simplement emmanchée sur l'extrémité de l'endoscope.

Toujours selon l'invention, l'endoscope 1 est pourvu de moyens de déplacement de la sonde 7 relativement au pied 10, en l'occurrence ici des actionneurs 13 (ici au nombre de trois répartis régulièrement à 120 degrés l'un de l'autre) qui s'étendent entre le pied 10 et la sonde 7 selon des directions sensiblement radiales concourantes pour permettre un déplacement commandé de la sonde 7 selon des directions transverses à l'axe longitudinal X de l'extrémité distale 4 de l'endoscope 1.

Ici, les actionneurs 13 sont symbolisés de façon schématique sous la forme d'actionneurs télescopiques articulés par leurs extrémités à la partie d'appui 11 du pied 10 et à la sonde 7. Il peut s'agir par exemple de vérins hydrauliques ou électromécaniques. La longueur des trois actionneurs 13 est ici variable pour déplacer la sonde en regard de l'organe interne. Pour cela, il convient de commander simultanément les trois actionneurs 13 pour déplacer la sonde 7 selon le trajet voulu.

L'utilisation de l'instrument endoscopique de l'invention est le suivant. On introduit l'endoscope 1 dans l'abdomen du patient de sorte à approcher le pied 10 de l'organe interne. On amène le pied en regard de la zone à examiner, et on applique la partie d'appui 11 du pied 10 contre l'organe interne avec une pression suffisante pour éviter tout mouvement de la partie d'appui 11 relativement à l'organe interne. On voit à la figure 3b que l'appui du pied 10 permet de déformer localement l'organe interne de sorte que la surface à examiner de celui-ci soit localement perpendiculaire à l'axe longitudinal X, ce qui n'est pas le cas avec un endoscope classique, comme illustré à la figure 3a.

L'appui du pied 10 sur l'organe interne étant réalisé, on commande alors les actionneurs 13 pour déplacer la sonde 7 de façon à réaliser un balayage de la zone à examiner. De préférence, le balayage est organisé de sorte que chacune des images prises par la sonde 7 recouvre la précédente image avec un taux de recouvrement d'environ 30%. Un logiciel de recomposition d'images récupère les images prises par la sonde 7 pour établir une cartographie de la zone examinée particulièrement précise. L'absence de mouvement de l'organe interne relativement au pied permet de garantir un excellent recouvrement des images prises par la sonde 7.

Selon maintenant un deuxième mode particulier de réalisation illustré à la figure 4a, la partie d'appui 11 du pied 10 est reliée à la partie de fixation 12 à l'endoscope 1 par une partie souple 14 permettant un mouvement relatif entre l'extrémité distale 4 de l'endoscope 1 et la partie d'appui 11 du pied 10. Cette disposition permet une certaine liberté de mouvement de l'organe d'interne relativement à l'endoscope 1. Cependant, l'application de la partie d'appui 11 du pied 10 sur l'organe interne empêche tout mouvement relatif de la partie d'appui 11 et de l'organe interne. Les actionneurs 13 s'étendant entre la partie d'appui 11 et la sonde 7, la position de la sonde 7 relativement à l'organe interne reste donc parfaitement contrôlée. Ainsi, la partie souple 14 permet la transmission d'un effort notamment longitudinal sur la partie d'appui 11 afin d'assurer que le pied 10 soit appuyé sur l'organe interne avec une pression suffisante pour empêcher tout mouvement relatif du pied 10 relativement à l'organe interne, tout en laissant la possibilité d'un mouvement notamment latéral de la partie d'appui 11 entraînée par l'organe interne lorsque celui-ci bouge. La partie souple 14 se déforme alors de sorte que l'extrémité distale 4 de l'endoscope 1 reste, elle, immobile par rapport à l'organe interne.

La partie souple 14 peut être venue de matière avec la partie d'appui 11 et la partie de fixation 12, par exemple sous la forme d'un soufflet. La partie souple 14 peut encore être un élément rapporté entre la partie d'appui 11 et la partie de fixation 12, par exemple un anneau élastomère. En variante, la partie souple peut s'étendre en amont du pied, en étant intégré à l'endoscope lui-même, le pied étant alors relié de façon rigide avec la partie souple à l'extrémité de l'endoscope.

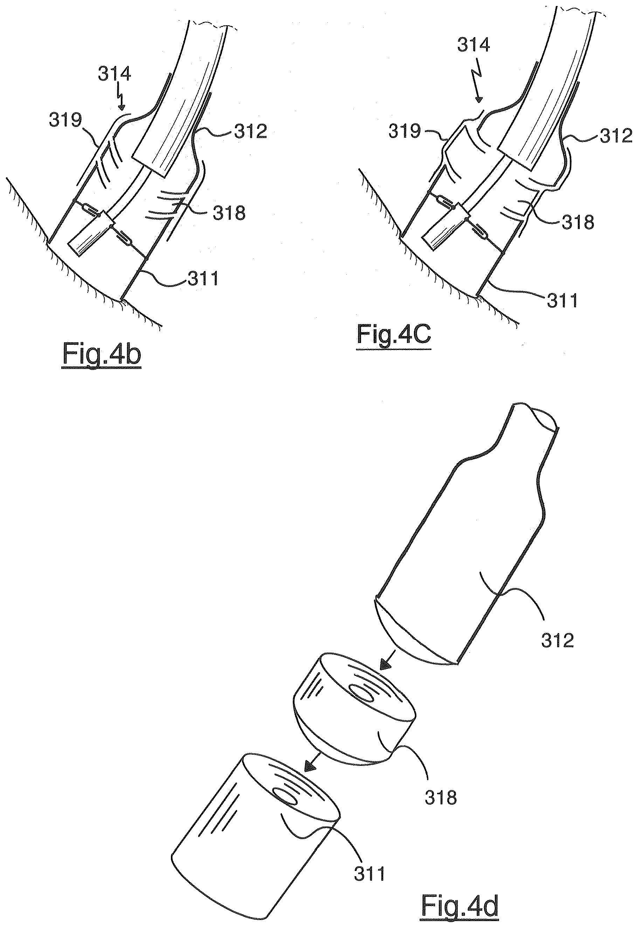

Selon une autre variante, en référence aux figures 4b et 4d, la partie souple 314 comporte au moins un premier élément de portée sphérique venu de matière avec la partie d'appui 311 et au moins un deuxième élément de portée sphérique homologue à celle du premier élément, le deuxième élément étant venu de matière avec la partie de fixation 312. La partie souple 314 comporte en outre une gaine 319 en matériau élastique recouvrant au moins en partie le premier élément et le deuxième élément. Les éléments sont bien entendu percés en leur centre pour permettre un passage de l'outil.

Par exemple, le premier élément a une portion sphérique concave et le deuxième élément a une portion sphérique convexe. Ici, la partie souple 314 comporte un troisième élément 318 interposé entre la partie d'appui 311 et la partie de fixation 312, le troisième élément 318 ayant des portées sphériques homologues à celles du premier élément et du deuxième élément.

Ainsi, la partie souple 314 permet la transmission d'un effort notamment longitudiè-nal sur la partie d'appui 311 afin d'assurer que le pied 310 soit appuyé sur l'organe interne avec une pression suffisante pour empêcher tout mouvement relatif du pied 310 relativement à l'organe interne tout en laissant la possibilité d'un mouvement notamment latéral de la partie d'appui 311 entraînée par l'organe interne lorsque celui-ci bouge. Comme illustré à la figure 4c, les éléments de la partie souple 314 se déplacent alors les uns par rapport aux autres de sorte que le deuxième élément et donc l'extrémité distale de l'endoscope reste immobile par rapport à l'organe interne.

Selon maintenant un troisième mode de réalisation illustré à la figure 5, les moyens de déplacement comportent deux séries d'actionneurs 13 et 13' s'étendant selon deux niveaux décalés selon l'axe longitudinal X, et permettant de contrôler finement l'orientation angulaire α de l'axe de travail Y de la sonde 7.

Ici, les axes des actionneurs sont concourants dans chacun des niveaux. On pourra bien sûr utiliser plus de deux niveaux d'actionneurs, par exemple si l'outil manipulé par ces actionneurs est flexible et qu'il faille le maintenir à plusieurs niveaux pour assurer un positionnement précis de l'extrémité de l'outil.

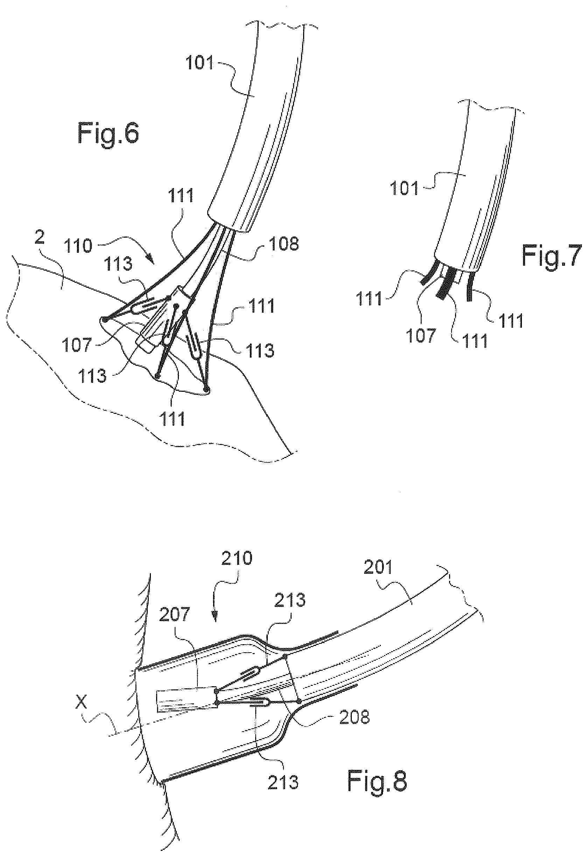

Selon maintenant un quatrième mode de réalisation illustré à la figure 6, le pied est maintenant sous la forme d'un trépied 110 comportant trois jambes 111 ayant des extrémités venant en appui contre l'organe interne et recevant chacune l'extrémité de l'un des actionneurs 113, qui s'étendent ici selon des directions concourantes.

De préférence, les jambes 111 sont flexibles et sont associées à des moyens de leur rétraction à l'intérieur de l'endoscope, pour aboutir à la configuration illustrée à la figure 7 dans laquelle le pied 110 est escamoté dans l'endoscope 101. Dans cette configuration, les actionneurs 113 sont rabattus les long des jambes 111.

Pour cela, il suffit par exemple de tirer sur le câble 108 de la sonde 107. Cette position escamotée facilite énormément l'introduction de l'endoscope dans le corps du patient. Après l'introduction et approche de l'extrémité distale de l'endoscope à proximité de l'organe interne, on provoque le déploiement du pied 110, ici en repoussant la sonde 107 via son câble 108 (si celui-ci est suffisamment rigide). Le pied 110 est alors prêt à être appliqué contre l'organe interne. Si le câble 108 n'est pas apte à assurer la fonction de déploiement/escamotage du pied 110, on prévoira alors des moyens d'actionnement spécifiques pour assurer ces opérations, par exemple une gaine tubulaire montée à l'intérieur de l'endoscope pour coulisser dans celui-ci, et à l'extrémité de laquelle les jambes 111 sont fixées.

Selon maintenant un cinquième mode particulier de réalisation de l'instrument endoscopique illustré à la figure 8, l'endoscope 201 porte un pied 210 et est équipé d'actionneurs 213 pour déplacer la sonde 207. Cependant, les actionneurs 213 s'étendent ici entre la sonde 207 et l'extrémité de l'endoscope 201.

Cette disposition permet d'imprimer plus facilement des mouvements à l'outil non plus seulement transversaux, mais également longitudinaux. Cette caractéristique est particulièrement utile dans le cas où l'outil est une aiguille à biopsie, ou encore si la sonde doit pénétrer dans l'organe interne.

L'invention n'est bien sûr pas limitée à ce qui vient d'être décrit, mais englobe au contraire toute variante entrant dans le cadre défini par les revendications. En particulier, les caractéristiques fonctionnelles décrites ici à l'occasion de la description des divers modes de réalisation de l'invention illustrés pourront bien sûr être combinées entre elles.

Le terme d'instrument endoscopique est à comprendre au sens large, et inclut les instruments suivants tels que bronchoscopes, gastroscopes, rectoscopes, laparoscopes, arthroscopes, ...

Bien qu'ici, l'outil porté par l'instrument endoscopique soit une sonde d'imagerie, l'invention n'est bien sûr pas limitée à ce type d'outil. L'invention s'applique également à un instrument endoscopique portant un outil de traitement (par exemple un outil d'abrasion ou de délivrance d'un médicament), un outil chirurgical (une aiguille, une lame, une pince...) ou tout autre outil.

Bien sûr, l'invention ne se limite pas à l'utilisation d'actionneurs télescopiques ni au nombre d'actionneurs mentionnés pour déplacer l'outil de l'instrument vis-à-vis du pied. Tout moyen de déplacement commmandable pourra être envisagé dans le cadre de l'invention, du moment qu'il permette des mouvements de l'outil selon au moins des directions transverses à un axe longitudinal de l'extrémité distale de l'instrument endoscopique. On pourra par exemple utiliser des actionneurs électromécaniques, hydrauliques, piezoélectriques, à base d'éléments en alliage à mémoire de forme. L'outil pourra encore être solidaire d'un manchon sensible à l'action d'un actionneur électromagnétique périphérique s'étendant autour du manchon et apte à attirer le manchon selon telle ou telle direction transverse.

Enfin, bien que la sonde soit ici reliée à l'extrémité distale de l'endoscope par son câble autorisant une liberté de mouvement de la sonde vis-à-vis du pied, on pourra bien entendu prévoir tout autre type de liaison de l'outil avec l'endoscope. Par exemple, l'outil peut être monté au bout d'un bras articulé à l'extrémité distale de l'endoscope. L'outil peut encore être solidarisé à l'endoscope uniquement par les moyens de déplacement.

Dans le quatrième mode de réalisation illustré à la figure 6, les jambes formant une partie d'appui du pied sont indépendantes les unes des autres. Bien entendu, en variante, les jambes pourront être reliées entre elles par exemple par un voile souple.

## Revendications

1. Instrument endoscopique (1) comportant un corps allongé présentant une extrémité distale (4) destinée à être introduite dans le corps du patient pour venir à proximité d'un organe interne, l'extrémité distale portant un outil (7) d'intervention sur l'organe interne, l'instrument endoscopique comportant un pied (10,110) solidaire de l'extrémité distale destiné à venir en appui sur l'organe interne, ainsi que des moyens de déplacement commandables (13,13',113) pour imprimer à l'outil des déplacements au moins selon des directions transverses à un axe longitudinal (X) de l'extrémité distale de l'instrument endoscopique alors que le pied est en appui contre l'organe interne, l'instrument endoscopique étant **caractérisé en ce que** les moyens de déplacement comportent au moins un actionneur (13, 13', 113, 213) qui est attelé à l'outil et qui est entièrement agencé dans le pied.

2. Instrument endoscopique selon la revendication 1, dans lequel l'actionneur (213) s'étend entre l'extrémité distale de l'instrument endoscopique et l'outil (207).

3. Instrument endoscopique selon la revendication 1, dans lequel l'actionneur s'étend entre le pied (10 ;110) et l'outil (7 ; 107).

4. Instrument endoscopique selon la revendication 1, dans lequel les moyens de déplacement comportent une pluralité d'actionneurs (13,13', 113) s'étendant selon des directions concourantes entre le pied et l'outil.

5. Instrument endoscopique selon la revendication 1, dans lequel les moyens de déplacement comportent au moins deux séries d'actionneurs (13,13') disposés selon des niveaux respectifs décalés pour s'étendre dans chacun des niveaux selon des directions concourantes entre le pied et l'outil.

6. Instrument endoscopique selon la revendication 1, dans lequel le pied comporte une partie d'appui (11) reliée à une partie de fixation (12) à l'endoscope, les parties d'appui et de fixation étant venues de matière.

7. Instrument endoscopique selon la revendication 1, dans lequel le pied comporte une partie d'appui (11) reliée à une partie de fixation (12) par une partie souple (14).

8. Instrument endoscopique selon la revendication 7, dans lequel la partie souple comporte :
- au moins un premier élément de portée sphérique venu de matière avec la partie d'appui (311) et au moins un deuxième élément de portée sphérique homologue à celle du premier élément, le deuxième élément étant venu de matière avec la partie de fixation (312) ;
- une gaine (319) en matériau élastique recouvrant au moins en partie le premier élément et le deuxième élément.

9. Instrument endoscopique selon la revendication 1, dans lequel le pied (110) comporte une pluralité de jambes (111) ayant des extrémités formant une partie d'appui du pied.

10. Instrument endoscopique selon la revendication 9, dans lequel au moins les extrémités des jambes formant une partie d'appui du pied sont indépendantes les unes des autres.

11. Instrument endoscopique selon la revendication 9, dans lequel les jambes (111) sont escamotables à l'intérieur de l'instrument endoscopique.

## Patentansprüche

1. Endoskopisches Instrument (1), umfassend einen länglichen Körper, der ein distales Ende (4) aufweist, das dazu bestimmt ist, in den Körper des Patienten eingeführt zu werden, um in die Nähe eines inneren Organs zu kommen, wobei das distale Ende ein Werkzeug (7) zum Eingriff an dem inneren Organ trägt, wobei das endoskopische Instrument einen fest mit dem distalen Ende verbundenen Fuß (10, 110) umfasst, der dazu bestimmt ist, an dem inneren Organ in Anlage zu kommen, sowie steuerbare Bewegungsmittel (13, 13', 113), um dem Werkzeug Bewegungen zumindest in Querrichtungen zu einer Längsachse (X) des distalen Endes des endoskopischen Instruments zu erteilen, während der Fuß in Anlage an dem inneren Organ ist, wobei das endoskopische Instrument **dadurch gekennzeichnet ist, dass** die Bewegungsmittel mindestens einen Aktor (13, 13', 113, 213) umfassen, der an das Werkzeug gekoppelt ist und der vollständig in dem Fuß angeordnet ist.

2. Endoskopisches Instrument nach Anspruch 1, bei dem sich der Aktor (213) zwischen dem distalen Ende des endoskopischen Instruments und dem Werkzeug (207) erstreckt.

3. Endoskopisches Instrument nach Anspruch 1, bei dem sich der Aktor zwischen dem Fuß (10; 110) und dem Werkzeug (7; 107) erstreckt.

4. Endoskopisches Instrument nach Anspruch 1, bei dem die Bewegungsmittel eine Vielzahl von Aktoren (13, 13', 113) umfassen, die sich in konvergenten Richtungen zwischen dem Fuß und dem Werkzeug erstrecken.

5. Endoskopisches Instrument nach Anspruch 1, bei dem die Bewegungsmittel mindestens zwei Reihen von Aktoren (13, 13') umfassen, die in entsprechenden versetzten Ebenen angeordnet sind, um sich in jeder der Ebenen in konvergenten Richtungen zwischen dem Fuß und dem Werkzeug zu erstrecken.

6. Endoskopisches Instrument nach Anspruch 1, bei dem der Fuß einen Stützteil (11) umfasst, der mit einem Befestigungsteil (12) zur Befestigung am Endoskop verbunden ist, wobei der Stützteil und der Befestigungsteil aus demselben Material integral geformt sind.

7. Endoskopisches Instrument nach Anspruch 1, bei dem der Fuß einen Stützteil (11) umfasst, der mit einem Befestigungsteil (12) über einen flexiblen Teil (14) verbunden ist.

8. Endoskopisches Instrument nach Anspruch 7, bei dem der flexible Teil umfasst:
- mindestens ein erstes Element mit kugelförmiger Auflagefläche, das aus demselben Material integral mit dem Stützteil (311) geformt ist, und mindestens ein zweites Element mit kugelförmiger Auflagefläche, die homolog zu der des ersten Elements ist, wobei das zweite Element aus demselben Material integral mit dem Befestigungsteil (312) geformt ist;
- eine Hülle (319) aus elastischem Material, die das erste Element und das zweite Element zumindest teilweise überdeckt.

9. Endoskopisches Instrument nach Anspruch 1, bei dem der Fuß (110) eine Vielzahl von Beinen (111) umfasst, die Enden haben, die einen Stützteil des Fußes bilden.

10. Endoskopisches Instrument nach Anspruch 9, bei dem mindestens die Enden der Beine, die einen Stützteil des Fußes bilden, voneinander unabhängig sind.

11. Endoskopisches Instrument nach Anspruch 9, bei dem die Beine (111) ins Innere des endoskopischen Instruments einziehbar sind.

## Claims

1. An endoscopic instrument (1) having an elongate body with a distal end (4) designed to be introduced into the body of the patient so as to come into proximity with an internal organ, the distal end carrying a tool (7) for intervention on the internal organ, the endoscopic instrument having a foot (10, 110) rigidly connected to the distal end and designed to bear on the internal organ, and also controllable means of movement (13, 13', 113) for conferring movements on the tool, at least in directions transverse to a longitudinal axis (X) of the distal end of the endoscopic instrument, when the foot is bearing against the internal organ, the endoscopic instrument being **characterized in that** the means of movement have at least one actuator (13, 13', 113, 213), which is coupled to the tool and which is arranged entirely within the foot.

2. The endoscopic instrument as claimed in claim 1, in which the actuator (213) extends between the distal end of the endoscopic instrument and the tool (207).

3. The endoscopic instrument as claimed in claim 1, in which the actuator extends between the foot (10; 110) and the tool (7; 107).

4. The endoscopic instrument as claimed in claim 1, in which the means of movement have a plurality of actuators (13, 13', 113) extending in convergent directions between the foot and the tool.

5. The endoscopic instrument as claimed in claim 1, in which the means of movement have at least two series of actuators (13, 13') arranged on respective offset levels, so as to extend, on each of the levels, in convergent directions between the foot and the tool.

6. The endoscopic instrument as claimed in claim 1, in which the foot has a bearing part (11) connected to a fixing part (12) for fixing to the endoscope, the bearing and fixing parts being made in one piece.

7. The endoscopic instrument as claimed in claim 1, in which the foot has a bearing part (11) connected to a fixing part (12) by a flexible part (14).

8. The endoscopic instrument as claimed in claim 7, in which the flexible part has:
- at least a first element providing a spherical support surface and made in one piece with the bearing part (311), and at least a second element providing a spherical support surface matching that of the first element, the second element being made in one piece with the fixing part (312);
- a sheath (319) made of elastic material and at least partly covering the first element and the second element.

9. The endoscopic instrument as claimed in claim 1, in which the foot (110) has a plurality of legs (111) with ends that form a bearing part of the foot.

10. The endoscopic instrument as claimed in claim 9, in which at least the ends of the legs forming a bearing part of the foot are independent of one another.

11. The endoscopic instrument as claimed in claim 9, in which the legs (111) are retractable inside the endoscopic instrument.
